# EUROPEAN PATENT SPECIFICATION

(11) **EP 2 175 834 B1**
(45) Date of publication and mention of the grant of the patent: **23.02.2011**
(21) Application number: 08772467.0
(22) Date of filing: 09.07.2008
(51) Int. Cl.: A61K 9/08, A61K 47/26, A61K 38/26

(54) **GLP-1-FC FUSION PROTEIN FORMULATION**
GLP-1-FC FUSIONSPROTEIN-FORMULIERUNG
FORMULATION DE PROTÉINE DE FUSION GLP-1-FC

(30) Priority: 10.07.2007 US 948855 P
(43) Date of publication of application: 21.04.2010
(73) Proprietor: ELI LILLY AND COMPANY, Indianapolis, IN 46285 (US)
(72) Inventor: NG, Kingman, Carmel, Indiana 46032 (US)
(74) Representative: Kent, Lindsey Ruth
(86) International application number: PCT/US2008/069473
(87) International publication number: WO 2009/009562

(56) References cited:
- WO-A-2006/068910

### SEQUENCE LISTING

<110> Eli Lilly and Company
<120> GLP-1-Fc FUSION PROTEIN FORMULATION
<130> X17652
<140> PCTUS0869473
   <141> 2008-07-09
<160> 1
<170> PatentIn version 3.5
<210> 1
   <211> 275
   <212> PRT
   <213> Artificial Sequence
<220>
   <223> Synthetic Construct
<400> 1
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Gly Gly
               20                  25                  30
   Gly Gly Gly Ser Gly Gly Gly Gly Ser Gly Gly Gly Gly Ser Ala Glu
           35                  40                  45
   Ser Lys Tyr Gly Pro Pro Cys Pro Pro Cys Pro Ala Pro Glu Ala Ala
       50                  55                  60
   Gly Gly Pro Ser Val Phe Leu Phe Pro Pro Lys Pro Lys Asp Thr Leu
   65                  70                  75                  80
   Met Ile Ser Arg Thr Pro Glu Val Thr Cys Val Val Val Asp Val Ser
                   85                  90                  95
   Gln Glu Asp Pro Glu Val Gln Phe Asn Trp Tyr Val Asp Gly Val Glu
               100                 105                 110
   Val His Asn Ala Lys Thr Lys Pro Arg Glu Glu Gln Phe Asn Ser Thr
           115                 120                 125
   Tyr Arg Val Val Ser Val Leu Thr Val Leu His Gln Asp Trp Leu Asn
       130                 135                 140
   Gly Lys Glu Tyr Lys Cys Lys Val Ser Asn Lys Gly Leu Pro Ser Ser
   145                 150                 155                 160
   Ile Glu Lys Thr Ile Ser Lys Ala Lys Gly Gln Pro Arg Glu Pro Gln
                   165                 170                 175
   Val Tyr Thr Leu Pro Pro Ser Gln Glu Glu Met Thr Lys Asn Gln Val
               180                 185                 190
   Ser Leu Thr Cys Leu Val Lys Gly Phe Tyr Pro Ser Asp Ile Ala Val
           195                 200                 205
   Glu Trp Glu Ser Asn Gly Gln Pro Glu Asn Asn Tyr Lys Thr Thr Pro
       210                 215                 220
   Pro Val Leu Asp Ser Asp Gly Ser Phe Phe Leu Tyr Ser Arg Leu Thr
   225                 230                 235                 240
   Val Asp Lys Ser Arg Trp Gln Glu Gly Asn Val Phe Ser Cys Ser Val
                   245                 250                 255
   Met His Glu Ala Leu His Asn His Tyr Thr Gln Lys Ser Leu Ser Leu
               260                 265                 270
   Ser Leu Gly
           275

## Description

### FIELD OF THE INVENTION

The present invention relates to a commercial formulation of a glucagon-like peptide analog fused to an Fc portion of an immunoglobulin. This formulation can be used to treat diabetes and obesity as well as a variety of other conditions or disorders.

### BACKGROUND OF THE INVENTION

Glucagon-like peptide-1 (GLP-1) analogs and derivatives show promise in clinical trials for the treatment of type 2 diabetes. GLP-1 induces numerous biological effects such as stimulating insulin secretion, inhibiting glucagon secretion, inhibiting gastric emptying, inhibiting gastric motility or intestinal motility, and inducing weight loss. A significant characteristic of GLP-1 is its ability to stimulate insulin secretion without the associated risk of hypoglycemia that is seen when using insulin therapy or some types of oral therapies that act by increasing insulin expression.

The usefulness of therapy involving GLP-1 peptides has been limited by the fact that GLP-1 (1-37) is poorly active, and the two naturally occurring truncated peptides, GLP-1(7-37)OH and GLP-1(7-36)NH₂, are rapidly cleared *in vivo* and have extremely short *in vivo* half lives. It is known that endogenously produced dipeptidyl-peptidase IV (DPP-IV) inactivates circulating GLP-1 peptides by removing the N-terminal histidine and alanine residues and is a major reason for the short *in vivo* half-life.

Various approaches have been undertaken to extend the elimination half-life of a GLP-1 peptide or reduce clearance of the peptide from the body while maintaining biological activity. One approach involves fusing a GLP-1 peptide to the Fc portion of an immunoglobulin. Immunoglobulins typically have long circulating half lives *in vivo.*

For example, IgG molecules can have a half-life in humans of up to 23 days. The Fc portion of the immunoglobulin is responsible, in part, for this *in vivo* stability. GLP-1-Fc fusion proteins take advantage of the stability provided by the Fc portion of an immunoglobulin while preserving the biological activity of the GLP-1 molecule.

Although this approach is feasible for GLP-1 therapeutics (See WO 02/46227), there is a general concern regarding the antigenicity of various fusion proteins when administered repeatedly over prolonged periods of time. This is especially a concern for GLP-1-Fc fusion therapeutics as a patient with diabetes must be treated for her entire life once diagnosed with the disease. In addition, Fc fusion protein therapeutics can be a concern if the Fc portion retains unwanted effector functions. This approach is the focus of PCT/US 04/15595 (WO2005/000892), in which problems associated with the potential immunogenicity and effector activity associated with administration of GLP-1-Fc fusion proteins are overcome by identifying specific GLP-1-Fc fusion proteins that have a reduced risk of inducing an immune response after repeated and prolonged administration and no longer have effector function.

The fusion proteins of this nature are technically too large and complex to produce synthetically or recombinantly in bacterial cells. These fusion proteins are typically produced in mammalian cells, such as CHO, 293, or NS0. It was observed that the fusion proteins produced in mammalian cells where more readily susceptible to degradation by endogenous proteases and chemical alteration than non-fusion proteins produced in bacterial cells. This problem was sought to be overcome in PCT/US 2005/045376 (WO2006/068910) wherein it was discovered that a formulation comprising a GLP-1-Fc fusion protein buffered between about pH 6 and about pH 8.5 provided increased chemical stability.

Yet, even when the instabilities caused by host cell proteases are held in check, the formulation may not be suitable if it is physically unstable. Another problem observed by the present inventor is the formation of soluble aggregates and insoluble particles upon long term storage of a solution formulation. This problem is sought to be overcome by a specific combination of excipients and a specific concentration of a GLP-1-Fc fusion protein.

### SUMMARY OF THE INVENTION

In order to overcome the problem of soluble aggregates and insoluble particles upon long term storage of a solution formulation of a GLP- 1-Fc fusion protein, the present inventor has developed a physically and chemically stable solution formulation comprising about 0.5 to about 10 mg/mL of a GLP-1-Fc fusion protein, 5 to 20 mM citrate buffer, 0.01 to 0.05% (w/v) polysorbate-80, and 4.0 to 5.3% (w/v) mannitol, and having a pH of 6-7. This formulation provided unexpectedly and considerably less soluble aggregates and insoluble particles upon long term storage. In addition, the present inventor discovered that this solution formulation is more stable in a syringe than in a vial after prolonged shelf storage.

The present invention also includes methods of treating patients suffering from diabetes and obesity was well as a variety of other conditions or disorders comprising administering the formulation of the GLP-1-Fc fusion protein.

### DETAILED DESCRIPTION OF THE INVENTION

The GLP-1-Fc fusion protein of the present invention comprises a GLP-1 compound fused at its C-terminus via a peptide linker to the N-terminus of an analog of an Fc portion of an immunoglobulin. The fusion protein is biologically active as a monomer or as a homodimer and has an increased half-life compared to native GLP-1. The preferred GLP-1-Fc fusion protein comprises the amino acid sequence given by (SEQ ID NO:1). The more preferred GLP-1-Fc fusion protein consists essentially of the amino acid sequence given by (SEQ ID NO:1). The most preferred GLP-1-Fc fusion protein consists of the amino acid sequence given by (SEQ ID NO:1).

Disulfide linkages can exist intra-chain (on either chain - A or B) and/or inter-chain (between both chains - A and B). Examples of intra chain disulfide linkages are: Cys90A - Cys150A, Cys196A - Cys254A, Cys90B - Cys150B, Cys196B - Cys254B. Examples of inter-chain disulfide linkages are: Cys55A - Cys55B, Cys58A - Cys58B.

Biological activity refers to the ability of the fusion protein to bind to and activate the GLP-1 receptor *in vivo* and elicit a response. Responses include, but are not limited to, secretion of insulin, suppression of glucagon, inhibition of appetite, weight loss, induction of satiety, inhibition of apoptosis, induction of pancreatic beta cell proliferation, and differentiation of pancreatic beta cells.

The GLP-1-Fc fusion protein formulation comprises about 0.25 to about 10 mg/ml of a GLP-1-Fc fusion protein. The preferred concentration of the fusion protein, in mg/mL, is in the range of about 0.5 to 10, 0.5 to 5, 0.5 to 2.5, 0.5 to 2, 0.5 to 1.67, 0.5 to 1.5, 0.5 to 1.25, 0.5 to 1, 0.5 to 0.9, 0.5 to 0.8,0.5 to 0.75, 0.6 to 2.0.7 to 2, 0.8 to 2, 0.9 to 2, 0.5 to 3, 0.6 to 3, 0.7 to 3, 0.8 to 3, 0.9 to 3, 0.5 to 4, 0.6 to 4, 0.7 to 4, 0.8 to 4, 0.9 to 4, 1 to 2, 1.1 to 2, 1.2 to 2, 1.3 to 2, 1.4 to 2, 1.5 to 2, 1.6 to 2, 0.7 to 1.67, 0.9 to 1.1, 1 to 4, 1.0 to 4.0, 0.5 to 5, 0.25 to 7, 0.25 to 5, 0.25 to 4, 0.25 to 3, 0.25 to 2, 0.25 to 1.5, 0.25 to 1, 0.25 to 0.5. The preferred concentration of the GLP-1-Fc fusion protein, in mg/mL, is about 0.25, about 0.42, about 0.5, about 0.6, about 0.67, about 0.7, about 0.75, about 0.8, about 0.83, about 0.9, about 1, about 1.1, about 1.2, about 1.25, about 1.3, about 1.4, about 1.5, about 1.6, about 1.67, about 1.7, about 1.8, about 1.9, about 2, about 2.5, about 3, about 3.33, about 4, about 5, about 6.67, or about 10.

The GLP-1-Fc fusion protein formulation is buffered in the range of about 5 to 20 mM citrate. The preferred citrate concentration, in mM, is in the range of about 5 to 15, 5 to 12.5, 5 to 10, 7.5 to 20, 7.5 to 15, 7.5 to 12.5, 7.5 to 10,8 to 20, 8 to 15, 8 to 12.5, 8 to 11, 8 to 10, 9 to 20, 9 to 15, 9 to 12.5, 10 to 20, 10 to 17.5, 10 to 15, 10 to 12.5, 6 to 14, 7 to 13, 8 to 12, 9 to 11, 12 to 20, 14 to 20, 16 to 20, and 18 to 20. The particularly preferred citrate concentration is in the range of about 9 to about 11, and about 8 to about 12 mM. The particularly preferred citrate concentration is about 10 or about 10.0.

The pH is adjusted in range of about 6 to 7 to provide acceptable stability, to maintain the solubility and insulinotropic activity of the GLP-1-Fc fusion protein and be acceptable for parenteral administration. The pH can be adjusted by adding acid, such as HCl, or base such as NaOH, to the desired pH or a combination of citrate buffer and citric acid can be added to achieve both the desired buffer concentration and the desired pH. The preferred pH value is in the range of about 6.3 to 6.7, 6.25 to 6.75, 6.2 to 6.8, 6.15 to 6.85, 6.1 to 6.9. The preferred pH value is about 6.5.

The GLP-1-Fe fusion protein formulation further comprises mannitol as an isotonicity agent. The mannitol concentration is in the range of 4.0 to 5.3% (w/v). The unit "(w/v)" means mass of the constituent per volume of the final formulation. Thus, a formulation having a mannitol concentration of 4.6% (w/v) has 46 mg of mannitol per mL of formulation, or expressed another way, it has 4.6 grams of mannitol dissolved in a total volume of 100 mL of formulation. The preferred mannitol concentration, in % (w/v) is in the range of about 4.0 to about 4. 1, about 4. 1 to about 4.2, about 4.2 to about 4.3, about 4.3 to about 4.4, about 4.4 to about 4.5, about 4.5 to about 4.6, about 4.6 to about 4.7, about 4.55 to about 4.75, about 4.5 to about 4.8, about 4.4 to about 4.9, about 4.3 to about 5.0, about 4.2 to about 5.1, about 4.1 to about 5.2, about 4.7 to about 4.8, about 4.8 to about 4.9, about 4.9 to about 5.0, about 5.0 to about 5.1, about 5.1 to about 5.2, about 5.2 to about 5.3. The preferred mannitol concentration, in % (w/v) is about 4.3, about 4.5, about 4.55, about 4.6, about 4.65, about 4.64, about 4.7, about 4.75, about 4.8, about 4.9, about 5.0, about 5.1, about 5.2, or about 5.3.

The GLP-1-Fc fusion protein formulation further comprises polysorbate-80 as a solubilizer and/or stabilizer. The concentration of polysorbate-80 is in the range of about 0.01 to 0.05% (w/v) (or expressed in terms of mg/ml, about 0.1 to 0.5 mg/mL). This concentration of polysorbate-80 was determined in combination with the GLP-Fc fusion protein and mannitol to minimize the formation of soluble aggregates and insoluble particles. The preferred concentration of polysorbate-80, in % (w/v) is in the range of about 0.01 to 0.04, 0.01 to 0.03, 0.015 to 0.025. A preferred concentration of polysorbate-80 is in the range of about 0.018 to about 0.022 % (w/v). Another preferred concentration of polysorbate-80 is in the range of about 0.015 to about 0.025 % (w/v). A particularly preferred concentration of polysorbate-80 is about 0.02% (w/v).

A particularly preferred formulation comprises the GLP-Fc fusion protein of having the amino acid sequence of SEQ ID NO: 1 in a concentration in the range of about 0.25 to about 10 mg/mL, citrate buffer in a concentration of about 10 mM, polysorbate-80 in a concentration of about 0.02% (w/v), mannitol in a concentration of about 4.6% (w/v), and a pH of about 6.5. Another particularly preferred formulation comprises the GLP-Fc fusion protein of having the amino acid sequence of SEQ ID NO: 1 in a concentration in the range of about 0.25 to about 5 mg/mL, citrate buffer in a concentration of about 10 mM, polysorbate-80 in a concentration of about 0.02% (w/v), mannitol in a concentration of about 4.6% (w/v), and a pH of about 6.5. Another particular formulation comprises the GLP-Fc fusion protein of having the amino acid sequence of SEQ ID NO: 1 in a concentration in the range of about 0.25 to about 10 mg/mL, citrate buffer in a concentration in the range of about 5 to about 20 mM, polysorbate-80 in a concentration of about 0.02% (w/v), mannitol in a concentration in the range of about 4.5 to about 4.8% (w/v), and a pH in the range of about 6.3 to about 6.7. Another particular formulation comprises the GLP-Fc fusion protein of having the amino acid sequence of SEQ ID NO:
1 in a concentration in the range of about 0.25 to about 5 mg/mL, citrate buffer in a concentration in the range of about 5 to about 20 mM, polysorbate-80 in a concentration of about 0.02% (w/v), mannitol in a concentration in the range of about 4.5 to about 4.8% (w/v), and a pH in the range of about 6.3 to about 6.7.

Administration of the formulations may be via any route known to be effective by the physician of ordinary skill. Peripheral parenteral is one such method. Parenteral administration is commonly understood in the medical literature as the injection of a dosage form into the body by a sterile syringe or some other mechanical device such as an infusion pump. Peripheral parenteral routes can include intravenous, intramuscular, subcutaneous, and intraperitoneal routes of administration. Subcutaneous administration is the preferred route.

The formulation of the present invention can be used to treat subjects with non-insulin dependent diabetes or at risk of developing non-insulin dependent diabetes, insulin dependent diabetes, or obesity. An effective amount of the GLP-1-Fc fusion protein in the context of the described formulation is the quantity which results in a desired therapeutic and/or prophylactic effect without causing unacceptable side-effects when administered to a subject in need of GLP-1 receptor stimulation.

It is preferable that the fusion proteins be administered either once every two weeks or once a week. Depending on the disease being treated, it may be necessary to administer the fusion protein more frequently such as two to three time per week.

The present invention will now be described only by way of non-limiting example with reference to the following Examples.

### EXAMPLES

### In vitro GLP-1 receptor activation assay

HEK-293 cells stably expressing the human GLP-1 receptor, using a CRE-Luciferase system, are seeded at 30,000 cells/well/80 µl low serum DMEM F 12 medium into 96 well plates. The day after seeding, 20 µl aliquots of test protein dissolved in 0.5% BSA are mixed and incubated with the cells for 5 hours. Generally 12 dilutions containing from 3 pM to 3 nM are prepared at a 5X concentration for each test protein before addition to the cells to generate a dose response curve from which EC₅₀ values are determined. After incubation, 100 µl of Luciferase reagent is added directly to each plate and mixed gently for 2 minutes. Plates are placed in a Tri-lux luminometer and light output resulting from luciferase expression is calculated.

### Analytical testing of GLP-Fc fusion formulation

GLP-Fc fusion formulation stability is assessed using the following methods: ultra violet-visible spectrometry (UV), reversed phase (RP) chromatography, size exclusion chromatography, anion exchange chromatography, limited digest with RP chromatography, absorbance at 550 nm, dynamic light scattering, instron, HIAC and differential scanning calorimetry (microDSC). Reversed-phase (RP) chromatography is used to monitor formation of clipped form GLP-Fc, oxidation in the Fc region and corresponding loss of intact main peak. Size exclusion (SE) HPLC is used to monitor polymer (soluble aggregate) formation and corresponding loss of monomer. Anion exchange (AEX) HPLC is used to monitor charge heterogeneity, particularly formation of acidic variants (AV), which usually corresponds to deamidation, and corresponding loss of main peak. Limited Digest is used to monitor degradation products specific to the peptide (GLP-1) portion of the GLP-Fc molecule, such as N-terminal clipping deletion of H1 (His at position 1 of SEQ ID NO:1) and/or G2 (Gly at position 2 of SEQ ID NO:1), protease clips at F22 (Phe at position 22 of SEQ ID NO: 1) and/or W25 (Trp at position 25 of SEQ ID NO:1), pyruvlation at the N-terminus, oxidation at W25 (Trp at position 25 of SEQ ID NO:1), and phosphorylation at S46 (Ser at position 46 of SEQ ID NO:1). Absorbance at 550 nm to monitor turbidity of the solution due to formation of insoluble particles. Dynamic light scattering is used to measure large soluble aggregate. Instron is used to measure filtration resistance, which is a semi - quantitative method initially developed to measure formation of gel - like structures in glucagon solution. This technique has been widely used to assess physical instability of GLP - 1 peptide solution. Since GLP-Fc fusion is a combination of a GLP - 1 analog with an IgG4 Fc chain, filtration resistance testing may provide further insight into the nature of the physical instability. The test is performed to measure back pressure from pushing the solution in syringe through a 13 mm diameter filter of PVDF membrane with 0.2 µm pore size. The pressure feedback graph has no slope if there is no resistance or aggregation. Increasing slopes over time indicate increasing amounts of aggregation and/or gelation. In order to simplify comparison of runs, only the maximum resistance values are reported here. HIAC is a light obstruction technique widely used in parenteral formulation development to monitor formation of insoluble particulate matters. Differential scanning calorimetry is used to monitor the unfolding characteristics as indicated by thermal transition temperature when the protein starts to undergo structural transition.

GLP-Fc fusion formulations are prepared according to the following table:

| Formulation GLP-Fc 1 mg/ml | pH | Buffer |
|---|---|---|
| 1 | 6 | 10 mM Citrate |
| 2 | 6.5 | 10 mM Citrate |
| 3 | 7 | 10 mM Citrate |
| 4 | 6 | 10 mM Histidine |
| 5 | 6.5 | 10 mM Histidine |
| 6 | 7 | 10 mM Phosphate |
| 7 | 7.5 | 10 mM Phosphate |
| 8 | 7.5 | 10 mM Tromethamine |
| 9 | 8 | 10 mM Tromethamine |

The GLP-Fc fusion formulations are sterile filtered through a 0.22 µm polyvinylidene fluoride (PVDF) membrane. The solutions are stored in 5 mL glass vials at 5, 15, 25, 37 and 45°C until analyzed or up to 20 weeks.

### Effects of pH on GLP-Fc stability:

The following table shows the rate constants for the formation of clipped forms of GLP-Fc fusion protein at 37°C as determined by RP chromatography.

| Formulation # | First order rate constant at 37 °C (week ⁻¹) |
|---|---|
| 1 | 4.84 E-03 |
| 2 | 5.22 E-03 |
| 3 | 7.01 E-03 |
| 4 | 3.83 E-03 |
| 5 | 5.05 E-03 |
| 6 | 1.28 E-02 |
| 7 | 1.85 E-02 |
| 8 | 6.43 E-03 |
| 9 | 8.89 E-03 |

The following table shows the rate constants for acidic variant formation of acidic variants of GLP-Fc fusion protein at 37°C as determined by AEX HPLC.

| Formulation # | First order rate constant at 37 °C (week⁻¹) |
|---|---|
| 1 | 4.25 E-02 |
| 2 | 4.05 E-02 |
| 3 | 4.73 E-02 |
| 4 | 2.36 E-02 |
| 5 | 4.18 E-02 |
| 6 | 4.31 E-02 |
| 7 | 5.06 E-02 |
| 8 | 5.27 E-02 |
| 9 | 6.46 E-02 |

The following table shows the formation of soluble aggregates (polymer %) of GLP-Fc fusion protein after 20 week storage at 37°C as determined by SE HPLC.

| Formulation # | Polymer % after 20 weeks at 37 °C |
|---|---|
| 1 | 4.6 |
| 2 | 2.4 |
| 3 | 1.4 |
| 4 | 2.6 |
| 5 | 1.4 |
| 6 | 2.2 |
| 7 | 1.1 |
| 8 | 0.6 |
| 9 | 0.3 |

The following table shows the rate constants for N-terminal clipping (des H1/H1G2) at 37 °C by limited digest.

| Formulation # | Zero order rate constant at 37 °C (% week⁻¹) |
|---|---|
| 1 | 0.42 |
| 2 | 0.32 |
| 3 | 0.32 |
| 4 | 0.39 |
| 5 | 0.36 |
| 6 | 0.43 |
| 7 | 0.52 |
| 8 | 0.31 |
| 9 | 0.29 |

### Solubility and viscosity

The following table shows the effect of pH on the solubility and viscosity of GLP-Fc fusion formulation. The solution is first prepared in the appropriate buffer, the pH is adjusted and then the solution is concentrated by centrifugation using a Centricon concentrator. The solution is checked visually for signs of reaching the solution's solubility limit. Concentration is determined by UV absorbance. Viscosity is measured in Poise (P) where 1 P = 1 g cm⁻¹ s⁻¹. Water at 20°C has a viscosity of approximately 0.01 g cm⁻¹ s⁻¹ which is the same as 1 centi-Poise (cP).

| Buffer | pH | Solubility (mg/mL) | Viscosity (cP) |
|---|---|---|---|
| 10 mM citrate | 5.5 | 27.1 | Not measured |
| 10 mM citrate | 5.8 | 146.2 | Not measured |
| 10 mM citrate | 6.0 | 179.4 | 8.3 |
| 10 mM citrate | 6.5 | 156.9 | 5.9 |
| 10 mM citrate | 7.0 | 158.1 | 7.7 |

### Solution formulation stability comparison

A Design of Experiment (DoE) study is set up to elucidate the relationship between key formulation parameters and chemical/physical stability properties of the molecule. Based on the data, a quantitative model is developed to (i) define an optimal target formulation with respect to chemical and physical stability properties; (ii) explore formulation design space to define parameter range for product with acceptable performance and (iii) establish the adequate robustness of the formulation performance within the design space explored in the study.

All the formulations tested in the DoE study are summarized in the following table. The various formulations are prepared and sterile filtered through a 0.22 µm polyvinylidene fluoride (PVDF) membrane. The formulations are stored in 3 mL glass vials at 5, 25 and 40°C until analyzed or up to 3 months.

| | Buffer | pH | GLP-Fc (mg/mL) | Polysorbate 80 (mg/mL) | Tonicity Agent Mannitol (50g/ml) NaCl (150mM) | EDTA (%) |
|---|---|---|---|---|---|---|
| 1 | 10 mM Citrate | 6.5 | 10 | 0.05 | Mannitol | 0 |
| 2 | 10 mM Citrate | 7.0 | 10 | 0.35 | NaCl | 0 |
| 3 | 10 mM Citrate | 7.0 | 10 | 0.05 | Mannitol | 0 |
| 4 | 10 mM Citrate | 7.0 | 10 | 0.35 | Mannitol | 0 |
| 5 | 10 mM Citrate | 7.0 | 10 | 0.05 | NaCl | 0 |
| 6 | 10 mM Citrate | 6.5 | 10 | 0.35 | Mannitol | 0 |
| 7 | 10 mM Citrate | 6.0 | 10 | 0.35 | NaCl | 0 |
| 8 | 10 mM Citrate | 6.0 | 10 | 0.05 | NaCl | 0 |
| 9 | 10 mM Citrate | 6.0 | 10 | 0.35 | Mannitol | 0 |
| 10 | 10 mM Citric | 6.5 | 10 | 0.2 | Mannitol | 0 |
| 11 | 10 mM Citrate | 7.0 | 10 | 0.2 | Mannitol | 0 |
| 12 | 10 mM Citrate | 6.0 | 10 | 0.2 | Mannitol | 0 |
| 13 | 10 mM Citrate | 6.5 | 10 | 0.2 | NaCl | 0 |
| 14 | 10 mM Citrate | 6.0 | 10 | 0.05 | Mannitol | 0 |
| 15 | 10 mM Citrate | 6.5 | 10 | 0.2 | Mannitol | 0 |
| 16 | 10 mM Citrate | 6.5 | 10 | 0.2 | NaCl | 0 |
| 17 | 10 mM Citrate | 6.5 | 10 | 0.2 | Mannitol | 0.01 |
| 18 | 10 mM Citrate | 6.5 | 10 | 0.2 | NaCl | 0.01 |
| 19 | 10 mM Histidine | 6.5 | 10 | 0.2 | Mannitol | 0.01 |
| 20 | 10 mM Histidine | 6.5 | 10 | 0.2 | NaCl | 0.01 |

The stability of formulations is assessed by monitoring decrease of main peak % by reversed phase (RP) chromatography. The rate constants for all formulations are shown in the following table.

| Formulation # | Zero order rate constant at 40 °C (% month⁻¹) |
|---|---|
| 1 | 7.65 |
| 2 | 9.78 |
| 3 | 9.08 |
| 4 | 8.44 |
| 5 | 9.06 |
| 6 | 8.16 |
| 7 | 7.41 |
| 8 | 6.90 |
| 9 | 7.03 |
| 10 | 6.98 |
| 11 | 8.31 |
| 12 | 7.10 |
| 13 | 7.42 |
| 14 | 7.97 |
| 15 | 7.41 |
| 16 | 7.30 |
| 17 | 4.92 |
| 18 | 5.52 |
| 19 | 5.69 |
| 20 | 5.52 |

There are two types of clip forms that can be monitored by RP chromatography. The first one is the clipped forms at F22 and/or W25 of the GLP region by residual proteases. The second type is clipped at the linker region via chemical mechanism. The rate constants for all formulations are shown in the following table as determined by RP chromotography.

| Fomulation # | Zero order rate constant at 40 °C (% month⁻¹) |
|---|---|
| 1 | 2.02 |
| 2 | 3.27 |
| 3 | 3.02 |
| 4 | 2.79 |
| 5 | 3.29 |
| 6 | 2.07 |
| 7 | 1.77 |
| 8 | 1.73 |
| 9 | 1.75 |
| 10 | 1.83 |
| 11 | 2.81 |
| 12 | 1.75 |
| 13 | 2.02 |
| 14 | 1.90 |
| 15 | 1.92 |
| 16 | 1.97 |
| 17 | 1.51 |
| 18 | 1.61 |
| 19 | 1.52 |
| 20 | 1.45 |

Soluble aggregate formation is monitored by size exclusion HPLC. The rate constants for monomer decrease at 40 °C are shown in the following table.

| Formulation # | Zero order rate constant at 40 °C (% month⁻¹) |
|---|---|
| 1 | 1.08 |
| 2 | 2.18 |
| 3 | 1.19 |
| 4 | 1.21 |
| 5 | 1.63 |
| 6 | 1.38 |
| 7 | 2.39 |
| 8 | 1.93 |
| 9 | 2.11 |
| 10 | 1.01 |
| 11 | 1.04 |
| 12 | 2.25 |
| 13 | 1.66 |
| 14 | 2.35 |
| 15 | 1.14 |
| 16 | 1.69 |
| 17 | 0.54 |
| 18 | 1.15 |
| 19 | 0.53 |
| 20 | 1.00 |

N-terminal clipping by limited digest within the GLP region is monitored by limited digest analysis. The rate constants for Des H1/H1G2 are shown in the following table.

| Formulation # | Zero order rate constant at 40 °C (% month ⁻¹) |
|---|---|
| 1 | 2.39 |
| 2 | 2.77 |
| 3 | 2.45 |
| 4 | 2.47 |
| 5 | 2.35 |
| 6 | 2.72 |
| 7 | 2.84 |
| 8 | 2.51 |
| 9 | 2.83 |
| 10 | 2.37 |
| 11 | 2.38 |
| 12 | 2.86 |
| 13 | 2.37 |
| 14 | 2.64 |
| 15 | 2.48 |
| 16 | 2.44 |
| 17 | 1.82 |
| 18 | 2.17 |
| 19 | 1.66 |
| 20 | 2.17 |

Soluble aggregation formation after agitation by orbital shaking at 400 RPM for 24 hours was monitored by SE HPLC. The monomer % results are shown in the following table.

| Formulation # | Monomer % |
|---|---|
| 1 | 94.5 |
| 2 | 98.0 |
| 3 | 96.1 |
| 4 | 98.1 |
| 5 | 97.8 |
| 6 | 98.2 |
| 7 | 97.9 |
| 8 | 97.3 |
| 9 | 98.0 |
| 10 | 98.0 |
| 11 | 98.2 |
| 12 | 97.9 |
| 13 | 98.1 |
| 14 | 94.4 |
| 15 | 98.1 |
| 16 | 98.1 |
| 17 | 98.2 |
| 18 | 98.1 |
| 19 | 98.5 |
| 20 | 98.3 |

A major concern for agitation stability is the formation of insoluble particulate matters, which can be monitored by HIAC measurements. The HIAC results after agitation by orbital shaking at 400 RPM for 24 hours are shown in the following table.

| Formulation # | HIAC 10 µm particle counts |
|---|---|
| 1 | 400 |
| 2 | 10 |
| 3 | 361 |
| 4 | 5 |
| 5 | 918 |
| 6 | 7 |
| 7 | 205 |
| 8 | 1251 |
| 9 | 17 |
| 10 | 4 |
| 11 | 10 |
| 12 | 26 |
| 13 | 29 |
| 14 | 1218 |
| 15 | 24 |
| 16 | 2 |
| 17 | 14 |
| 18 | 16 |
| 19 | 9 |
| 20 | 2 |

### Comparison of NaCl vs. Mannitol formulation in Syringe and Vial

| Formulation | Composition |
|---|---|
| 1 | 1 mg/mL GLP-Fc, 10 mM Citrate pH 6.5, 150 mM NaCl, 0.02% (w/v) Polysorbate 80 |
| 2 | 1 mg/mL GLP-Fc, 10 mM Citrate pH 6.5, 5% (w/v) mannitol, 0.02% (w/v) Polysorbate 80 |

Stability comparison at 5° C of the above two formulations comparing mannitol and NaCl and syringe and vial storage.

| 5C | Main Peak % | | | |
|---|---|---|---|---|
| Time (mo) | Formulation 1 Syringe | Formulation 2 Syringe | Formulation 1 Vial | Formulation 2 Vial |
| 0 | 77.4 | 77.1 | 77.4 | 77.1 |
| I | ND | ND | 77.1 | 77.4 |
| 3 | 76.9 | 77.2 | 76.7 | 76.5 |
| 6 | 76.4 | 76.4 | 75.8 | 75.9 |
| | | | | |

| 5C | Clipped % | | | |
|---|---|---|---|---|
| Time (mo) | Formulation 1 Syringe | Formulation 2 Syringe | Formulation 1 Vial | Formulation 2 Vial |
| 0 | 0.3 | 0.2 | 0.3 | 0.2 |
| I | ND | ND | 0.3 | 0.2 |
| 3 | 0.3 | 0.2 | 0.2 | 0.3 |
| 6 | 0.4 | 0.4 | 0.4 | 0.4 |
| | | | | |

| 5C | Monomer % | | | |
|---|---|---|---|---|
| Time (mo) | Formulation 1 Syringe | Formulation 2 Syringe | Formulation 1 Vial | Formulation 2 Vial |
| 0 | 98.1 | 97.1 | 98.1 | 97.1 |
| 1 | 96.6 | 95.3 | ND | ND |
| 3 | 97.9 | 97.9 | 97.6 | 97.8 |
| 6 | 97.7 | 98 | 96.6 | 97.1 |

| | | | | |
|---|---|---|---|---|
| ND = Not Determined | | | | |

Stability comparison at 25° C of the above two formulations comparing mannitol and NaCl and syringe and vial storage

| 25C | Main Peak % | | | |
|---|---|---|---|---|
| Time (mo) | Formulation 1 Syringe | Formulation 2 Syringe | Formulation 1 Vial | Formulation 2 Vial |
| 0 | 77.4 | 77.1 | 77.4 | 77.1 |
| 1 | ND | ND | 72.9 | 73.5 |
| 3 | 64.0 | 68.0 | 53.8 | 64.1 |
| 6 | 46.7 | 59.6 | 39.3 | 41.9 |
| | | | | |

| 25C | Clipped % | | | |
|---|---|---|---|---|
| Time (mo) | Formulation 1 Syringe | Formulation 2 Syringe | Formulation 1 Vial | Formulation 2 Vial |
| 0 | 0.3 | 0.2 | 0.3 | 0.2 |
| 1 | ND | ND | 1.0 | 0.8 |
| 3 | 2.4 | 1.8 | 4.9 | 2.5 |
| 6 | 7.1 | 3.4 | 10.1 | 9.1 |
| | | | | |

| 25C | Monomer % | | | |
|---|---|---|---|---|
| Time (mo) | Formulation 1 Syringe | Formulation 2 Syringe | Formulation 1 Vial | Formulation 2 Vial |
| 0 | 98.1 | 97.1 | 98.1 | 97.1 |
| 1 | 95.0 | 95.0 | ND | ND |
| 3 | 91.8 | 94.1 | 85.4 | 92.9 |
| 6 | 82.2 | 91.7 | 76 | 81.7 |

| | | | | |
|---|---|---|---|---|
| ND = Not Determined | | | | |

Stability comparison at 40° C of the above two formulations comparing mannitol and NaCl and syringe and vial storage

| 40C | Main Peak % | | | |
|---|---|---|---|---|
| Time (mo) | Formulation 1 Syringe | Formulation 2 Syringe | Formulation 1 Vial | Formulation 2 Vial |
| 0 | 77.4 | 77.1 | 77.4 | 77.1 |
| 1 | ND | ND | 37.6 | 39 |
| 3 | 24.2 | 30.2 | 24.4 | 22.5 |
| | | | | |

| 40C | Clipped % | | | |
|---|---|---|---|---|
| Time (mo) | Formulation 1 Syringe | Formulation 2 Syringe | Formulation 1 Vial | Formulation 2 Vial |
| 0 | 0.3 | 0.2 | 0.3 | 0.2 |
| 1 | ND | ND | 10.5 | 9.7 |
| 3 | 17.3 | 14.9 | 17.1 | 16.8 |
| | | | | |

| 40C | Monomer % | | | |
|---|---|---|---|---|
| Time (mo) | Formulation 1 Syringe | Formulation 2 Syringe | Formulation 1 Vial | Formulation 2 Vial |
| 0 | 98.1 | 97.1 | 98.1 | 97.1 |
| 1 | 86.3 | 91.3 | ND | ND |
| 3 | 82.2 | 86.5 | 80.5 | 85.2 |

| | | | | |
|---|---|---|---|---|
| ND = Not Determined | | | | |

### Mannitol concentration determination

The mannitol concentration needed to achieve the target tonicity of 290 milli-Osmolarity/Kg for a GLP-Fc fusion protein solution formulation is determined by titration experiment. The following table summarizes the resulted osmolality as a function of mannitol concentration. Based on linear regression analysis of the osmolality results to mannitol concentration with a statistical p value of < 0.01, the mannitol concentration is determined to be 46.4 mg/mL or 4.64%.

| Volume (mL) 10 mM citrate butler | Volume (mL) 10 mM citrate/buffer with 50 mg/mL mannitol | Final Mannitol Concentration (mg/mL) | Osmolality (mOsm/Kg) | | |
|---|---|---|---|---|---|
| | | | Rep1 | Rep2 | Ave. |
| 1.0 | 0 | 0 | 34 | 34 | 34 |
| 0.2 | 9.8 | 49.0 | 308 | 302 | 305 |
| 0.4 | 9.6 | 48.0 | 300 | 304 | 302 |
| 0.5 | 9.5 | 47.5 | 301 | 301 | 301 |
| 0.7 | 9.3 | 46.5 | 293 | 291 | 292 |
| 0.8 | 9.2 | 46.0 | 284 | 284 | 284 |
| 0.9 | 9.1 | 45.5 | 283 | 286 | 285 |
| 1.0 | 9.0 | 45.0 | 280 | 281 | 281 |

## Claims

1. A stable solution formulation comprising a therapeutically effective amount of a GLP-1-Fc fusion protein in citrate buffer with polysorbate-80 in the range of 0.01% to 0.05% (w/v), mannitol in the range of 4.3 to 5.0% (w/v), and wherein the solution has a pH in the range of pH 6 to 7.

2. The stable solution formulation of claim 1, wherein the therapeutically effective amount of a GLP-1-Fc fusion protein is in the range of 0.25 to 10 mg/ml.

3. The stable solution formulation of claim 2, wherein the therapeutically effective amount of a GLP-1-Fc fusion protein is in the range of 0.25 to 5 mg/ml.

4. The stable solution formulation of any one of claims 1 to 3, wherein the concentration of citrate buffer is in the range of 5 to 20 mM.

5. The stable solution formulation of claim 4, wherein the concentration of citrate buffer is about 10 mM.

6. The stable solution formulation of any one of claims 1 to 5, wherein the concentration of polysorbate-80 is 0.02% (w/v).

7. The stable solution formulation of any one of claims 1 to 6, wherein the concentration of mannitol is in the range of 4.5 to 4.8% (w/v).

8. The stable solution formulation of any one of claims 1 to 7, wherein the concentration of mannitol is about 4.6% (w/v).

9. The stable solution formulation of any one of claims 1 to 8, wherein the amino acid sequence of the GLP-1-Fc fusion protein is that given by SEQ ID NO: 1

10. The stable solution formulation of any one of claims 1 to 9, wherein the concentration of the GLP-1-Fc fusion protein is in the range of about 0.25 to 5 mg/mL, the concentration of citrate is about 10 mM, the concentration of polysorbate-80 is about 0.02% (w/v), the concentration of mannitol is about 4.6% (w/v) and the pH is in the range of 6.3 to 6.7.

11. The stable solution formulation of any one of claims 1 to 10, wherein the concentration of GLP-1-Fc fusion protein is about 1 mg/mL.

12. The stable solution formulation of any one of claims 1 to 11, wherein the pH is about 6.5.

13. The stable solution formulation of any one of claims 1 to 12, wherein the formulation is stored in a sterile syringe.

14. A stable solution formulation according to any one of the preceding claims for use in the treatment of non-insulin dependent diabetes.

15. A stable solution formulation according to any one of claims 1 to 13 for use in the treatment of obesity.

## Patentansprüche

1. Formulierung in Form einer stabilen Lösung, die eine therapeutisch wirksame Menge eines GLP-1-Fc-Fusionsproteins in Citratpuffer mit Polysorbat-80 in einem Bereich von 0,01 % bis 0,05 % (g/v), Mannit in einem Bereich von 4.3 bis 5,0 % (g/v) umfasst, wobei die Lösung einen pH-Wert im Bereich von 6 bis 7 aufweist.

2. Formulierung in Form einer stabilen Lösung nach Anspruch 1, wobei die therapeutisch wirksame Menge eines GLP-1-Fc-Fusionsproteins in einem Bereich von 0,25 bis 10 mg/ml liegt.

3. Formulierung in Form einer stabilen Lösung nach Anspruch 2, wobei die therapeutisch wirksame Menge eines GLP-1-Fc-Fusionsproteins in einem Bereich von 0,25 bis 5 mg/ml liegt.

4. Formulierung in Form einer stabilen Lösung nach einem der Ansprüche 1 bis 3, wobei die Konzentration des Citratpuffers in einem Bereich von 5 bis 20 mM liegt.

5. Formulierung in Form einer stabilen Lösung nach Anspruch 4, wobei die Konzentration des Citratpuffers etwa 10 mM beträgt.

6. Formulierung in Form einer stabilen Lösung nach einem der Ansprüche 1 bis 5, wobei die Konzentration von Polysorbat-80 0.02 % (g/v) beträgt.

7. Formulierung in Form einer stabilen Lösung nach einem der Ansprüche 1 bis 6, wobei die Konzentration von Mannit in einem Bereich von 4,5 bis 4,8 % (g/v) liegt.

8. Formulierung in Form einer stabilen Lösung nach einem der Ansprüche 1 bis 7, wobei die Konzentration von Mannit etwa 4,6 % (g/v) beträgt.

9. Formulierung in Form einer stabilen Lösung nach einem der Ansprüche 1 bis 8, wobei die Aminosäuresequenz des GLP-1-Fc-Fusionsproteins die durch die SEQ ID NO: 1 angegebene ist.

10. Formulierung in Form einer stabilen Lösung nach einem der Ansprüche 1 bis 9, wobei die Konzentration des GLP-1-Fc-Fusionsproteins in einem Bereich von etwa 0.25 bis 5 mg/ml liegt, die Konzentration des Citrats etwa 10 mM beträgt, die Konzentration von Polysorbat-80 etwa 0,02 % (g/v) beträgt, die Konzentration von Mannit etwa 4,6 % (g/v) beträgt und der pH-Wert in einem Bereich von 6,3 bis 6,7 liegt.

11. Formulierung in Form einer stabilen Lösung nach einem der Ansprüche 1 bis 10, wobei die Konzentration des GLP-1-Fc-Fusionsproteins etwa 1 mg/ml beträgt.

12. Formulierung in Form einer stabilen Lösung nach einem der Ansprüche 1 bis 11, wobei der pH-Wert etwa 6,5 beträgt.

13. Formulierung in Form einer stabilen Lösung nach einem der Ansprüche 1 bis 12, wobei die Formulierung in einer sterilen Spritze aufbewahrt ist.

14. Formulierung in Form einer stabilen Lösung nach einem der vorhergehenden Ansprüche zur Verwendung bei der Behandlung von nicht-insulinabhängigem Diabetes.

15. Formulierung in Form einer stabilen Lösung nach einem der Ansprüche 1 bis 13 zur Verwendung bei der Behandlung von Fettleibigkeit.

## Revendications

1. Formulation sous forme de solution stable comprenant une quantité thérapeutiquement efficace d'une protéine de fusion GLP-1-Fc dans du tampon de type citrate avec du polysorbate-80 dans la plage de 0,01 % à 0,05 % (pds/vol), de mannitol dans la plage de 4,3 à 5,0% (pds/vol), et dans laquelle la solution a un pH situé dans la plage de pH 6 à 7.

2. Formulation sous forme de solution stable selon la revendication 1, dans laquelle la quantité thérapeutiquement efficace d'une protéine de fusion GP-1-Fc se situe dans la plage de 0,25 à 10 mg/ml.

3. Formulation sous forme de solution stable selon la revendication 2, dans laquelle la quantité thérapeutiquement efficace d'une protéine de fusion GLP-1-Fc se situe dans la plage de 0,25 à 5 mg/ml.

4. Formulation sous forme de solution stable selon l'une quelconque des revendications 1 à 3, dans laquelle la concentration du tampon citrate se situe dans la plage de 5 à 20 mM.

5. Formulation sous forme de solution stable selon la revendication 4, dans laquelle la concentration du tampon citrate est d'environ 10 mM.

6. Formulation sous forme de solution stable selon l'une quelconque des revendications 1 à 5, dans laquelle la concentration du polysorbate-80 est de 0,02 % (pds/vol).

7. Formulation sous forme de solution stable selon l'une quelconque des revendications 1 à 6, dans laquelle la concentration du mannitol est située dans la plage de 4,5 à 4,8 % (pds/vol).

8. Formulation sous forme de solution stable selon l'une quelconque des revendications 1 à 7, dans laquelle la concentration du mannitol est d'environ 4,6 % (pds/vol).

9. Formulation sous forme de solution stable selon l'une quelconque des revendications 1 à 8, dans laquelle la séquence d'acides aminés de la protéine de fusion GLP-1-Fc est celle donnée par SEQ ID NO : 1.

10. Formulation sous forme de solution stable selon l'une quelconque des revendications 1 à 9, dans laquelle la concentration de la protéine de fusion GLP-1-Fc est située dans la plage d'environ 0,25 à 5 mg/ml, la concentration du citrate est d'environ 10 mM, la concentration du polysorbate-80 est d'environ 0,02 % (pds/vol), la concentration du mannitol est d'environ 4,6 % (pds/vol) et le pH se situe dans la plage de 6,3 à 6,7.

11. Formulation sous forme de solution stable selon l'une quelconque des revendications 1 à 10, dans laquelle la concentration de la protéine de fusion GLP-1-Fc est d'environ 1 mg/ml.

12. Formulation sous forme de solution stable selon l'une quelconque des revendications 1 à 11, dans laquelle le pH est d'environ 6,5.

13. Formulation sous forme de solution stable selon l'une quelconque des revendications 1 à 12, dans laquelle la formulation est stockée dans une seringue stérile.

14. Formulation sous forme de solution stable selon l'une quelconque des revendications précédentes pour l'utilisation dans le traitement du diabète non insulino dépendant.

15. Formulation sous forme de solution stable selon l'une quelconque des revendications 1 à 13, pour l'utilisation dans le traitement de l'obésité.
